# EUROPÄISCHE PATENTSCHRIFT

(11) **EP 3 813 731 B1**
(45) Veröffentlichungstag und Bekanntmachung des Hinweises auf die Patenterteilung: **11.02.2026**
(21) Anmeldenummer: 19739208.7
(22) Anmeldetag: 25.06.2019
(51) Int. Cl.: A61F 2/07, B33Y 80/00, B33Y 10/00

(54) **STENTGRAFT UND VERFAHREN ZU DESSEN HERSTELLUNG**
STENT GRAFT AND METHOD FOR PRODUCING SAME
ENDOPROTHÈSE COUVERTE ET PROCÉDÉ POUR SA FABRICATION

(30) Priorität: 26.06.2018 DE 102018005070
(43) Veröffentlichungstag der Anmeldung: 05.05.2021
(73) Patentinhaber: Rheinisch-Westfälische Technische Hochschule (RWTH) Aachen, 52062 Aachen (DE)
(72) Erfinder: LÖWEN, Alexander, Aachen 52064 (DE); GESCHÉ, Valentine, Aachen 52070 (DE); JOCKENHÖVEL, Stefan, Aachen 52072 (DE); GRIES, Thomas, Aachen 52072 (DE)
(74) Vertreter: Vossius & Partner Patentanwälte Rechtsanwälte mbB
(86) Internationale Anmeldenummer: PCT/EP2019/066906
(87) Internationale Veröffentlichungsnummer: WO 2020/002371

(56) Entgegenhaltungen:
- WO-A1-2016/116748
- US-A1- 2007 293 936
- US-A1- 2008 082 160
- US-A1- 2013 296 998
- US-A1- 2013 331 927
- US-A1- 2015 320 956

## Beschreibung

Die vorliegende Erfindung betrifft einen Stentgraft sowie ein Verfahren zu dessen Herstellung, wobei insbesondere eine Stentstruktur mittels eines additiven Verfahrens auf einen Graft aufgebracht wird.

Gefäße, insbesondere Blutgefäße, können sich auf Grund zahlreicher Faktoren, einschließlich Alterung, Ernährung, Stress oder Krankheit mit der Zeit verändern. Besonders risikobehaftet sind hierbei Änderungen in Bezug auf die Elastizität und die Stabilität der Gefäßwände, die sich oft auf den Durchmesser der Gefäße auswirken, sowie Änderungen in Bezug auf die Funktion von, als Ventile wirkender, Klappen.

Um die von Gefäßveränderungen ausgehende Gefahr zu verringern, sind verschiedene Möglichkeiten bekannt. Häufig wird auf Gefäßimplantate zurückgegriffen, die den entsprechenden Gefäßabschnitt überbrücken oder auch ersetzen. Beispielsweise kann ein lokal verengtes Gefäß durch einen Bypass ersetzt werden oder ein krankhaft erweitertes Gefäß durch einen Stentgraft überbrückt beziehungsweise ersetzt werden.

Stentgrafts sind Hohlzylinder mit einer Längserstreckung und weisen einen tubulären Graft und eine in der Regel metallene Stentstruktur zur Stabilisierung des Grafts auf. Stentstruktur und Graft werden üblicherweise in getrennten Fertigungsprozessen hergestellt und anschließend durch zum Teil noch manuelle Schritte relativ zeit- und kostenintensiv assembliert, beispielsweise durch Vernähen. Beispiele für derartige Gefäßimplantate sind beispielsweise aus der GB 2 347 861 A, der US 2004/193258 A1 oder auch der US 5 911 753 A bekannt, und erhältliche Produkte finden bereits routinemäßige Anwendung.

Hinsichtlich der individuellen Gefäßgeometrie sind jedoch neben klinisch etablierten Standardprodukten patientenindividualisierte Lösungen wünschenswert. Diese sind allerdings noch immer mit vergleichsweise hohen Herstellungskosten und zeitintensiven Produktionsschritten verbunden.

Ein Verfahren zur Erstellung einer Gefäßstütze basierend auf einem digitalen 3D Modell ist aus der DE 10 2015 207 596 A1 bekannt. Dabei wird eine Vielzahl von Schichten insbesondere mittels eines (Poly-jet) Verfahrens aufeinander aufgetragen. Dass den vielfältigen klinischen Anforderungen an eine solche Gefäßstütze auf diese Weise genügt werden kann, scheint jedoch fraglich.

Darüber hinaus ist die Herstellung eines hybriden Polymerstents aus der US 2015/0335451 A1 bekannt. Auch diese Stents haben sich in der Praxis jedoch offenbar nicht durchgesetzt.

Weitere Beispiele für Stentgrafts sowie deren Herstellung sind im Folgenden aufgeführt, wobei gemäß dem Stand der Technik beispielsweise die Stentstruktur mittels eines additiven Verfahrens hergestellt sein kann.

So stellt US 2007/061004 A1 einen ein Lumen stützenden Stent mit einem lichten Durchgangslumen zur Verwendung in einem Körperlumen bereit. Besagter Stent ist aus mindestens einer Reihe von Gleit- und Verriegelungsradialelementen und mindestens einem Ratschenmechanismus gebildet, der ein Gelenkelement und mehrere Anschläge umfasst. Der Ratschenmechanismus ermöglicht dabei ein Einweg-Gleiten der Radialelemente von einem komprimierten Durchmesser in einen expandierten Durchmesser, verhindert jedoch einen radialen Rückstoß aus dem expandierten Durchmesser.

US 2009/043330 A1 stellt einen PreStent bereit, der eine Hülle und einen Rahmen umfasst, die zum Vorbereiten eines Gefäßdurchgangs für die anschließende Zuführung eines Stents ausgebildet sind, wobei der PreStent selbstexpandierend oder ballonexpandierbar sein kann. Ferner wird ein Stützsystem für die sichere Zuhführung des PreStents bereitgestellt, wobei das Stützsystem einen Zuführungskatheter, einen oder mehrere Okklusionsballons, optional einen oder mehrere Dilatationsballons und eine Retentionshülle für den selbstexpandierenden PreStent-Typ umfasst.

US 2017/333133 A1 stellt ein Verfahren zum Erzeugen einer patientenspezifischen Prothese bereit, welches das Empfangen anatomischer Bildgebungsdaten, die für einen Teil der Anatomie eines Patienten repräsentativ sind, umfasst. Ferner wird eine erste digitale Darstellung der anatomischen Bildgebungsdaten definiert, diese modifiziert, und darauf basierend eine zweite digitale Darstellung des Teils der Anatomie des Patienten definiert. Eine patientenspezifische Prothesenschablone des Teils der Anatomie des Patienten wird dann zumindest teilweise auf Grundlage der zweiten digitalen Darstellung der anatomischen Bildgebungsdaten erzeugt.

US 2012/197382 A1 stellt eine endoluminale Prothese bereit, welche ein Transplantat mit einem röhrenförmigen Körper mit einem proximalen und einem distalen Ende und einen Klappenersatz, der zwischen dem proximalen und dem distalen Ende des Transplantats angeordnet ist, umfasst. Mindestens ein Stent ist mit dem Transplantat verbunden und weist einen komprimierten Abgabezustand und einen expandierten Zustand zur Aufrechterhaltung der Durchgängigkeit innerhalb eines Teils des Transplantats auf.

US 2007/293936 A1 beschreibt Verfahren zur Herstellung individueller endovaskulärer Stents und Stentgrafts.

Vor diesem Hintergrund beabsichtigt die vorliegende Erfindung, ein automatisierbares, zeit- und kosteneffizientes Verfahren bereit zu stellen, das die Herstellung von Stentgrafts und insbesondere von individualisierten Stentgrafts vereinfacht. Die Erfindung beabsichtigt zudem, dass die hergestellten Stentgrafts eine hohe Biokompatibilität aufweisen und den Anforderungen in der klinischen Praxis genügen. Hierzu zählen unter anderem eine ausreichende Crimpbarkeit und/oder Faltbarkeit zur endovaskulären Einführung und/oder eine ausreichende radiale Steifigkeit in einem expandierten Zustand.

Erfindungsgemäß wird dies durch ein Verfahren und einen Stentgraft gemäß den beigefügten Ansprüchen erreicht, wobei bevorzugte Weiterbildungen in den abhängigen Ansprüchen aufgeführt sind.

Der erfindungsgemäße Stentgraft weist einen Graft und eine Stentstruktur auf, die mittels eines additiven Verfahrens vorzugsweise direkt auf den Graft aufgebracht ist. Dabei ist die Stentstruktur vorzugsweise polymerbasiert und/oder aus einem polymeren Material hergestellt.

Das erfindungsgemäße Verfahren zur Herstellung des Stentgrafts umfasst das Bereitstellen eines Grafts, der vorzugsweise aus einem ersten polymerbasierten Material hergestellt ist, und das Aufbringen einer Stentstruktur mit einer Mehrzahl von Streben aus einem zweiten polymerbasierten Material auf den Graft mittels eines additiven Verfahrens, bevorzugt mittels Fused Deposition Modeling. Der Graft und die aufgebrachte Stentstruktur sind dabei vorzugsweise derart ausgebildet, dass der Stentgraft in mindestens einem komprimierten Zustand und in mindestens einem expandierten Zustand angeordnet werden kann, wobei der Stentgraft in dem mindestens einen komprimierten Zustand einen geringeren Querschnitt als in dem mindestens einen expandierten Zustand aufweist.

Somit wird gemäß dem erfindungsgemäßen Verfahren nicht nur, wie bereits aus dem Stand der Technik bekannt, die Stentstruktur mittels eines additiven Verfahrens hergestellt, sondern vielmehr die Stentstruktur mittels eines additiven Verfahrens, bevorzugt mittels Fused Deposition Modeling, direkt auf den Graft aufgebracht. So kann die Herstellung, insbesondere auch individualisierter, Stentgrafts automatisiert, zeit- und kosteneffizient erfolgen und sichergestellt werden, dass der jeweilig hergestellte, bevorzugt metalllose, Stentgraft den Anforderungen in der klinischen Praxis besonders gut genügt. So können insbesondere aus dem Stand der Technik bekannte Methoden zum Aufbringen einer Stentstruktur auf einen Graft wie Nähen, Kleben oder Verschweißen vermieden werden.

Im Kontext der vorliegenden Erfindung bezieht sich der Begriff "Stentgraft" auf ein endovaskuläres Gefäßimplantat oder eine endovaskuläre Gefäßprothese, die mindestens eine Stentstruktur und mindestens einen daran befestigten Graft aufweist. Der Begriff "Stentstruktur" bezeichnet hierbei eine Struktur, die vorzugsweise eine Mehrzahl von Streben aufweist. Die Stentstruktur ist vorzugsweise tubulär und/oder tubusförmig. Der Begriff "Graft" bezeichnet eine Struktur, welche ein Medium innerhalb eines Gefäßlumens leitet. Der Graft ist vorzugsweise mit einer Stentstruktur verbunden, beispielsweise in Form einer Auskleidung und/oder Ummantelung der Stentstruktur. Eine solche Ummantelung ist vorzugsweise tubulär und/oder tubusförmig. Die Ummantelung ist vorzugsweise aus einem polymerbasierten und/oder polymeren Material hergestellt.

Der hier verwendete Begriff "Stentgraft" bezeichnet vorzugsweise eine Stentstruktur mit einem daran befestigten Graft und umfasst somit jede Art endovaskulärer Gefäßimplantate, die zumindest temporär mindestens eine vorzugsweise tubuläre Struktur mit einer Mehrzahl von Streben und mindestens eine vorzugsweise polymerbasierte Materialschicht als Ummantelungskomponente aufweisen.

Im Kontext der vorliegenden Erfindung bezeichnet der Begriff "Gefäß" jede Art tubulärer Körperstruktur, insbesondere des menschlichen Körpers, die mindestens ein Fluid wie beispielsweise ein Gas und/oder eine Flüssigkeit transportiert, nicht ausschließlich aber insbesondere Hohlgefäße wie beispielsweise Blutgefäße. Das Gefäß kann dabei in verschiedenen Körperregionen lokalisiert sein, beispielsweise in den Extremitäten oder im Gehirn, insbesondere aber im Abdomen und/oder Thorax. Als betroffenes Gefäß wird im Folgenden ein Gefäß mit einer genetisch bedingten und/oder erworbenen Gefäßwandveränderung bezeichnet. Der Ausdruck betroffener Bereich bezieht sich auf den lokal begrenzten Bereich der Gefäßwandveränderung in dem betroffenen Gefäß sowie unmittelbar daran angrenzende Bereiche. Als intakt werden im Folgenden Zustände, Geometrie und/oder Eigenschaften bezeichnet, die das Gefäß in dem betroffenen Bereich ohne Gefäßwandveränderung unter Berücksichtigung verschiedener Faktoren, wie beispielsweise Alter, Geschlecht und/oder Körpergewicht aufweisen würde und/oder aufgewiesen hat. Hierbei können beispielsweise auch Zustände, Geometrie und/oder Eigenschaften der unmittelbar an den Bereich der Gefäßwandänderung angrenzenden Gefäßbereiche berücksichtigt werden. Ferner werden als Aneurysmen Veränderungen einer Gefäßwand bezeichnet, wobei verschiedene Definitionen des Begriffs "Aneurysma" dem Fachmann bekannt sind. So können beispielsweise Veränderungen einer Gefäßwand als Aneurysma bezeichnet werden, die zu einer Vergrößerung des Durchmessers des betroffenen Gefäßes führen, im Allgemeinen zu einer Vergrößerung um mindestens 50 % im Vergleich zu dem Durchmesser eines intakten Gefäßes in dem jeweiligen Bereich. Auch der absolute Durchmesser eines Gefäßes in dem Bereich der Gefäßwandveränderung kann als Indikation für ein Aneurysma herangezogen werden, beispielsweise im Falle einer abdominalen Aorta von 5 cm bis 5,5 cm bei Männern beziehungsweise von 4,5 cm bis 5 cm bei Frauen. Auch im Falle anderer Gefäße wie beispielsweise thorakaler oder thorako-abdominaler Aorten können entsprechende Werte, möglicherweise mit geringfügiger Abweichung, als Indikation für ein Aneurysma herangezogen werden. Gegebenenfalls kann die Zunahme des Gefäßdurchmessers auch geringer sein, insbesondere wenn die Veränderung vergleichsweise rasch voranschreitet. So kann beispielsweise die Zunahme des Gefäßdurchmessers von mindestens 5 mm innerhalb von 6 Monaten auf ein erhöhtes Rupturrisiko bzw. auf ein Aneurysma des betroffenen Gefäßes hinweisen.

Der Stentgraft kann zur Behandlung von Aneurysmen ausgebildet sein. Insbesondere kann es sich bei den erfindungsgemäßen Stentgrafts um Stentgrafts zur Behandlung von Aortenaneurysmen handeln, wie etwa zur Behandlung von Aneurysmen der thorakalen, thorako-abdominalen oder abdominalen Aorta.

Erfindungsgemäß wird die mindestens eine Stentstruktur auf die mindestens eine Materialschicht des Grafts mittels eines additiven Verfahrens aufgebracht. Im Rahmen der vorliegenden Erfindung beinhaltet der Begriff "additives Verfahren" dem Fachmann bekannte additive Fertigungsverfahren beziehungsweise Verfahren zur additiven oder generativen Fertigung, bei denen Elemente durch Auf- und/oder Aneinanderfügen von Material automatisiert hergestellt werden. Additive Verfahren umfassen beispielsweise 3D-Druck Verfahren sowie Schmelzschichtungsverfahren (z.B. Fused Deposition Modeling). Beim Fused Deposition Modeling wird das bevorzugt polymerbasierte Material der Stentstruktur beispielsweise durch eine beheizte Düse verflüssigt und in Form von Filamenten in einer oder mehreren Schichten auf den Graft aufgebracht, vorzugsweise unmittelbar und/oder direkt auf den Graft. Das Material kann der Düse als Filament zugeführt werden. Alternativ kann das Material als Granulat vorliegen, welches mittels eines Extruders aufgeschmolzen wird und der Düse zugeführt wird.

Der bereitgestellte Graft ist vorzugsweise tubulär und/oder tubusförmig, wobei der Graft trichterförmig sein und/oder Durchmessersprünge aufweisen kann und/oder Verzweigungen/ Abzweigungen aufweisen kann. Bevorzugt ist der Graft zylindrisch. Die Stentstruktur wird somit vorzugsweise auf einen zylindrischen Graft aufgebracht. Der Graft könnte jedoch auch als Materialschicht bereitgestellt werden, z. B. als eine ebene Materialschicht. In diesem Fall könnten nach dem Aufbringen der Stentstruktur der Graft mit der darauf aufgebrachten Stentstruktur in eine tubuläre Form gebracht werden, beispielsweise durch Nähen, Kleben, Verschweißen oder Verschmelzen.

Das Aufbringen der Stentstruktur auf den Graft erfolgt bevorzugt durch den Einsatz eines rotierbaren Halters, mittels dem der Graft gehalten und gedreht werden kann, vorzugsweise um seine Längsachse. Bei einem solchen Halter kann es sich beispielsweise um einen rotierenden tubulären Kern und/oder ein rotierendes tubuläres Gerüst handeln, auf dessen Außenfläche der bereitgestellte Graft aufliegt. In diesem Fall wird die mindestens eine Stentstruktur bevorzugt außen auf den Graft aufgebracht. Sofern die aufgebrachte Stentstruktur innerhalb des Grafts angeordnet werden soll, kann das erfindungsgemäße Verfahren einen weiteren, optionalen Schritt des Umstülpens des Stentgrafts umfassen. Der Graft des erfindungsgemäßen Stentgrafts kann daher so vorgesehen werden, dass er die Stentstruktur von innen oder außen ummantelt.

Ferner kann der erfindungsgemäße Stentgraft mindestens eine Stentstruktur aufweisen, die zumindest teilweise innen und außen auf dem Graft angeordnet ist. Optional oder alternativ kann der erfindungsgemäße Stentgraft mindestens eine Stentstruktur aufweisen, die auf einer Innenseite des Grafts angeordnet ist, und mindestens eine Stentstruktur, die auf einer Außenseite des Grafts angeordnet ist. Jeweilige Stentstrukturen können auf einem Graft dabei so angeordnet sein, dass ein jeweiliger Bereich des erfindungsgemäßen Stentgrafts, der mindestens eine Stentstruktur auf der Innenseite des Grafts aufweist, zugleich mindestens eine Stentstruktur auf der Außenseite des Grafts aufweist. Alternativ können jeweilige Stentstrukturen auf dem Graft so angeordnet sein, dass mindestens ein Bereich des erfindungsgemäßen Stentgrafts, der mindestens eine Stentstruktur auf einer Innenseite des Grafts aufweist, zugleich mindestens eine Stentstruktur auf einer Außenseite des Grafts aufweist. Ferner können alternativ jeweilige Stentstrukturen auf dem Graft so angeordnet sein, dass ein jeweiliger Bereich des erfindungsgemäßen Stentgrafts, der eine Stentstruktur auf einer Innenseite des Grafts aufweist, keine Stentstruktur auf einer Außenseite des Grafts aufweist. Die jeweiligen Stenstrukturen, die auf einer Innenseite und/oder auf einer Außenseite des Stentgrafts angeordnet sind, überlappen somit in der Längsrichtung des Stentgrafts vorzugsweise nicht oder nur teilweise. Bevorzugt weist der erfindungsgemäße Stentgraft mindestens eine Stentstruktur auf, die in mindestens einem Bereich eines tubulären Endes des Stentgrafts auf dessen Innenseite angeordnet ist, und mindestens eine Stentstruktur, die in mindestens einem mittleren Bereich des tubulären Stentgrafts auf dessen Außenseite angeordnet ist.

Derartige Stentgrafts können erfindungsgemäß beispielsweise hergestellt werden, indem mindestens eine erste Stentstruktur auf einen Graft aufgebracht wird, bevorzugt durch den Einsatz eines rotierbaren Halters wie ebenfalls zuvor beschrieben. Das erfindungsgemäße Verfahren weist bevorzugt ferner einen Schritt des Umstülpens des Grafts mit der mindestens ersten aufgebrachten Stentstruktur auf, gefolgt von mindestens einem weiteren Schritt des Aufbringens mindestens einer weiteren Stentstruktur wie zuvor beschrieben, wobei die jeweilige mindestens eine Stentstruktur in einem oder mehreren Bereichen des Grafts angeordnet sein kann oder diesen über die gesamte Länge und/oder den gesamten Umfang umgeben kann.

Der Graft ist vorzugsweise polymerbasiert. Im Kontext der vorliegenden Erfindung umfasst der Begriff "polymerbasiert" sowohl die Ausdrücke "mindestens ein Polymer aufweisend", "bestehend aus einem Anteil an mindestens einem Polymer", wobei der Anteil bis zu 50 % oder bis zu 70 %, bevorzugt bis zu 75 % oder bis zu 80 % und besonders bevorzugt bis zu 90 % oder bis zu 95 % ausmacht, als auch "gefertigt aus mindestens einem Polymer" beziehungsweise "bestehend zu 100 % aus mindestens einem Polymer".

Alternativ oder zusätzlich ist der Graft vorzugsweise ein Textil und/oder textilbasiert. Zur Erstellung eines solchen Textils können dem Fachmann bekannte Techniken und Materialen verwendet werden, wobei der bereitgestellte Graft bevorzugt aus biokompatiblen und/oder hämokompatiblen Materialien hergestellt ist, wie etwa Polytetrafluorethylen (PTFE), Polyethylenterephthalat (PET), Polyvinylidenfluorid (PVDF), Polyurethan (PU), Polylactid (PLA) und/oder Polycaprolacton (PCL). Alternativ oder zusätzlich ist der bereitgestellte Graft bevorzugt als Gewebe und/oder Maschenware ausgebildet. Dabei wird unter einem Gewebe vorzugsweise Webware verstanden, die durch das rechtwinklige Verkreuzen von mindestens zwei Fadensystemen hergestellt wird, als Maschenware hingegen Strick- und Wirkware, die einen oder mehrere verschlungene Fäden aufweist. Für die Herstellung des Grafts können beispielsweise Monofilament- und/oder Multifilamentgarne als Fäden verwendet werden. In Bezug auf den Gesamttiter des Fadens können die Fäden beispielsweise gemäß EN ISO 2060:1995, bevorzugt nach der unter Punkt 4 Prinzip genannten Variante 1 oder für Monofilamente beispielsweise gemäß EN 13392:2001 eine Feinheit von 1 dtex bis 200 dtex, vorzugsweise von 10 dtex bis 60 dtex haben. Der Graft kann dabei zum Beispiel nach seiner Herstellung eine Materialschichtdicke von 0,01 mm bis 2 mm, vorzugsweise von 0,1 mm bis 0,5 mm aufweisen, vorzugsweise optisch gemessen am Stentgraft.

Der bereitgestellte Graft weist vorzugsweise eine Maschenware auf und/oder ist aus einer solchen Maschenware ausgebildet.

Diese Maschenware kann insbesondere mittels der Doppelrascheltechnik hergestellt sein, wobei es sich vorzugsweise um eine Jacquard-Technik handelt. Diese erlaubt eine besonders flexible Anpassung des Grafts an die jeweilige Patientenanatomie.

Der bereitgestellte Graft weist vorzugsweise eine Vielzahl von Poren auf, beispielsweise mehr als 100 oder mehr als 1000 Poren, die durch Fadenzwischenräume des Textils gebildet werden, insbesondere durch die Maschen zwischen dem/den Faden/Fäden, wobei der/die jeweilige/n Faden/Fäden durch mehrere Filamente gebildet sein können. Die Größe und die Öffnungsfläche der Poren sind daher gut einstellbar. Vorzugsweise weist der Graft eine Maschen- bzw. Porengröße von 1 µm bis 1000 µm auf, stärker bevorzugt von 10 µm bis 300 µm. Die einzelnen Poren können daher eine Öffnungsfläche von 1 bis 1.000.000 µm², vorzugsweise von 100 bis 90.000 µm², aufweisen.

Die Poren des Grafts können gleich und/oder verschieden groß sein und beispielsweise auch in ihrer Größe in Strömungsrichtung entlang des Stentgrafts variieren. Dies kann beispielsweise eine lagefeste Positionierung des Grafts im Gefäß durch Einwachsen von Gewebe in die Poren unterstützen.

Der bereitgestellte Graft kann flüssigkeitsdurchlässig ausgestaltet sein. In diesem Fall bildet der Graft vorzugsweise ein selbstdichtendes System, wobei die in dem Gefäß transportierte Flüssigkeit den Graft nach Implantation des Stentgrafts abdichtet, beispielsweise durch Koagulation in der Flüssigkeit enthaltener Bestandteile in und/oder an dem Graft. Zu diesem Zweck können die Porengrößen so gewählt sein, dass sie bei Implantierung des Stentgrafts in einem Blutgefäß beispielsweise durch eine Ablagerung von Fibrin im Rahmen der Blutgerinnung am Graft abgedichtet werden können. Der bereitgestellte Graft kann jedoch auch flüssigkeitsundurchlässig sein, etwa indem der Graft mit Kollagen und/oder Gelatine beschichtet ist. Eine jeweilige Beschichtung kann dabei auf einen Graft aufgebracht werden, bevor eine Stentstruktur auf den jeweiligen Graft aufgebracht wird. In diesem Fall weist das erfindungsgemäße Verfahren einen Schritt einer Beschichtung des Grafts vor dem Aufbringen einer Stentstruktur auf den Graft auf. Alternativ kann eine jeweilige Beschichtung auf einen Graft aufgebracht werden, nachdem eine Stentstruktur auf diesen aufgebracht wurde. In diesem Fall weist das erfindungsgemäße Verfahren nach dem Aufbringen einer Stentstruktur auf den Graft einen Schritt einer Beschichtung des hergestellten Stentgrafts auf.

Die Stentstruktur kann den Graft stützen und ist vorzugsweise komprimierbar, um den Stentgraft in dem mindestens einen komprimierten Zustand anzuordnen, und/oder expandierbar, um den Stentgraft in dem mindestens einen expandierten Zustand anzuordnen. Zu diesem Zweck kann die Stentstruktur elastisch verformbar sein und/oder mindestens ein Smart Polymer aufweisen. Alternativ oder zusätzlich kann die Stentstruktur mechanisch expandierbar sein, etwa über einen Ballonkatheter.

Die Streben der Stentstruktur können auf verschiedene Ausgestaltungsformen angeordnet sein, wobei sie vorzugsweise eine kompressible Helikale-, Zickzack- oder Maschenstruktur ausbilden, optional und/oder alternativ auch mäanderförmige Ringe (Kronen). Zur Kompression der Stentstruktur kann hierbei vorzugsweise der Öffnungswinkel zwischen zwei an einem Verbindungs- und/oder Kreuzpunkt miteinander verbundenen Streben verringert werden. Eine entsprechende Kompression der Stentstruktur kann auch als "Crimpen" bezeichnet werden.

Als Smart Polymer oder auch smarte Polymere werden im Kontext der vorliegenden Erfindung Polymere bezeichnet, die sich durch ihre Sensitivität gegenüber mindestens einem externen Faktor, wie beispielsweise Temperatur, Feuchtigkeit, pH Wert, Lichtintensität, elektrischen Feldern und/oder Magnetfeldern auszeichnen und als Reaktion auf mindestens einen dieser externen Faktoren mindestens eine physikalische Eigenschaft, bevorzugt reversibel und bevorzugt schnell ändern. Bevorzugte smarte Polymere sind beispielsweise Co-Block Polymere, die unter Umständen auch zur Wirkstofffreisetzung genutzt werden können.

Vorzugsweise weist die Stentstruktur mindestens ein Shape Memory Polymer auf. Shape Memory Polymere haben als smarte Polymere insbesondere die Eigenschaft, ihre Form als Reaktion auf mindestens einen der genannten externen Faktoren auf eine vordefinierte Art zu ändern. Beispiele für bevorzugte Shape Memory Polymere sind auf Polyurethan (PU)-basierende Polymere. Vorzugsweise wird ein Shape Memory Polymer verwendet, das bei Erreichen einer bestimmten Temperatur in eine zuvor antrainierte Form zurückspringt, beispielsweise bei einer Temperatur von über 30 °C, über 35 °C, über 37 °C, über 40 °C oder über 45°C (beispielsweise Yakacki et al., Biomaterials, 2007, 28:2255-63). Die Rückverformung in die antrainierte Form wird vorzugsweise bei einer Temperatur von unter 55 °C, unter 50° C, unter 45° C oder unter 40 °C erreicht.

Die Stentstruktur kann zusätzlich oder alternativ zu dem mindestens einen Smart Polymer mindestens ein nicht-smartes, biokompatibles Polymer aufweisen und/oder mindestens ein Additiv aufweisen. Additive bezeichnen im Rahmen der vorliegenden Erfindung dem Fachmann bekannte Zusatzstoffe, die die Eigenschaften des verwendeten Polymers modifizieren, beispielsweise organische Füllstoffe, z.B. Kohlenstoff-Fasern, oder anorganische Füllstoffe, z.B. Silikate. Nicht-smarte, biokompatible Polymere bezeichnen im Rahmen der vorliegenden Erfindung dem Fachmann bekannte Polymere, die zur Verwendung als Implantate geeignet sind, insbesondere Polyethylenterephthalat (PET), Polytetrafluorethylen (PTFE), Polyvinylidenfluorid (PVDF) und Polyetheretherketon (PEEK) und Thermoplastisches Polyurethan (TPU). Durch die Wahl des mindestens einen für die Stentstruktur verwendeten polymerbasierten Materials kann die Kompressibilität und die radiale Steifigkeit im expandierten Zustand des hergestellten Stentgrafts angepasst werden. Das Polymer kann zudem derart gewählt werden, dass abhängig vom gewählten additiven Fertigungsverfahren und dem Material des Grafts eine ausreichende Haftung zwischen Stentstruktur und Graft erreicht wird. Alternativ oder zusätzlich kann der Graft temperiert werden und/oder der Bauraum des 3D-Druckers temperiert werden, um eine verbesserte Haftung zwischen Stentstruktur und Graft zu erzielen. Die eingestellte Temperatur kann z.B. im Bereich der Glasübergangstemperatur des aufgedruckten Polymers liegen. Auch eine Kombination von Polymeren mit unterschiedlichen Materialeigenschaften ist möglich. Eine solche Kombination kann beispielsweise durch Compoundieren bereitgestellt werden. Alternativ und/oder zusätzlich können Polymere mit unterschiedlichen Materialeigenschaften im Rahmen der additiven Fertigung kombiniert werden, beispielsweise durch Aufdrucken verschiedener Schichten, Nutzung mehrerer Düsen und/oder Verwendung von Bikomponentendüsen. Das Material und/oder die Verarbeitungsbedingungen (etwa die Temperatur) kann/können so gewählt sein, dass das Material der Stentstruktur zumindest teilweise in die Poren des Grafts hineinfließt und/oder von einer Seite des Grafts zur anderen hindurchfließt. Alternativ oder zusätzlich kann/können das Material und/oder dessen Verarbeitungsbedingungen (etwa die Temperatur) so gewählt sein, dass zumindest einige Fäden des Grafts im Material der Stentstruktur eingebettet sind und/oder von dem Material der Stentstruktur über Abschnitte der Fadenlänge vollständig umschlossen werden.

Die Ausgestaltung der Stentstruktur ist mittels additiver Fertigung frei wählbar und individuell gestaltbar, insbesondere im Fall des Fused Deposition Modeling. Die aufzuschmelzenden Filamente können hierbei zum Beispiel einen Durchmesser von 0,5 mm bis 3,5 mm aufweisen, bevorzugt von 1 mm bis 3 mm, und besonders bevorzugt von 1,75 mm bis 2,85 mm.

Die Stentstruktur weist vorzugsweise eine Mehrzahl von miteinander verbundenen Streben auf, wobei die Streben in einem Querschnitt senkrecht zur Verlaufsrichtung der jeweiligen Strebe in Radialrichtung des Stentgrafts eine Höhe von mindestens 0,01 mm, vorzugsweise mindestens 0,05 mm oder mindestens 0,1 mm haben. So können die Streben zum Beispiel eine Höhe von 0,01 mm bis 3 mm, bevorzugt von 0,05 mm bis 1 mm aufweisen. Alternativ oder zusätzlich können die Streben in diesem Querschnitt senkrecht zur Höhe eine Breite von mindestens 0,01, vorzugsweise mindestens 0,05 mm oder 0,1 mm haben. So können die Streben zum Beispiel eine Breite von 0,01 mm bis 5 mm, bevorzugt von 0,01 mm bis 3 mm, besonders bevorzugt von 0,05 mm bis 1 mm aufweisen.

Die Streben können in einem Querschnitt senkrecht zur Verlaufsrichtung der jeweiligen Strebe in Radialrichtung des Stentgrafts (Höhenrichtung der jeweiligen Strebe) aus mehreren Lagen aufgebaut sein. Die Dicke einer einzelnen Lage kann 0,005 mm bis 1 mm, bevorzugt 0,01 mm bis 0,7 mm und besonders bevorzugt 0,02 mm bis 0,4 mm betragen. Alternativ oder zusätzlich können die Streben in einem Querschnitt senkrecht zur Verlaufsrichtung der jeweiligen Strebe senkrecht zur Radialrichtung des Stentgrafts (Breitenrichtung der jeweiligen Strebe) aus mehreren Schichten aufgebaut sein, z.B. aus mehreren nebeneinander extrudierten Linien. Die Extrusionsbreite einer einzelnen Linie kann 0,01 mm bis 5 mm, bevorzugt 0,05 mm bis 2 mm und besonders bevorzugt 0,1 mm bis 1 mm oder 0,1 mm bis 0,5 mm betragen. Alternativ oder zusätzlich können sich die einzelnen extrudierten Linien in Breitenrichtung überlappen, beispielsweise um höchstens 70 %, bevorzugt um höchstens 50 % und besonders bevorzugt um höchstens 30 %.

Die Streben können in dem Fachmann bekannten Ausgestaltungsformen angeordnet sein, beispielsweise in zickzack- und/oder mäanderförmigen Ringen (Kronen), spiralförmig und/oder in zickzackförmigen Spiralen. Die Spiralen und/oder Ringe können ihrerseits über Verbinder miteinander verbunden sein. Die Streben können so vorgesehen sein, dass sie im expandierten Zustand höchstens 50 %, höchstens 40 %, höchstens 30 %, höchstens 20 %, höchstens 12 %, höchstens 10 %, höchstens 8 %, höchstens 6 %, höchstens 5 % oder höchstens 3 % der inneren Mantelfläche des Grafts und/oder höchstens 50 %, höchstens 40 %, höchstens 30 %, höchstens 20 %, höchstens 12 %, höchstens 10 %, höchstens 8 %, höchstens 6 %, höchstens 5 % oder höchstens 3 % der äußeren Mantelfläche des Grafts bedecken. Ferner können die Streben so vorgesehen sein, dass sie im expandierten Zustand mindestens 12 %, mindestens 10 %, mindestens 8 %, mindestens 5 %, mindestens 3 % oder mindestens 1 % der inneren Mantelfläche des Grafts und/oder mindestens 12 %, mindestens 10 %, mindestens 8 %, mindestens 5 %, mindestens 3 % oder mindestens 1 % der äußeren Mantelfläche des Grafts bedecken.

Bei der bevorzugten Verwendung des Fused Depositon Modeling kann die Stentstruktur ferner eine Struktur aufweisen, bei der Strukturelemente wie beispielsweise Windungen ohne Überkreuzungen der Filamente ausgebildet sind. Ferner weist die Stentstruktur vorzugsweise keine nachträglich induzierten, lokalen Änderungen der Materialeigenschaften auf, wie beispielsweise Nähte, Schweißzonen und/oder Kleberückstände.

Der erfindungsgemäße Stentgraft ist vorzugsweise derart ausgestaltet, dass er den von einer Veränderung betroffenen Bereich stützen, überbrücken und/oder ersetzen kann. So weist der Stentgraft vorzugsweise eine ausreichende strukturelle Stabilität und Elastizität auf, um die Funktionsfähigkeit des Gefäßimplantats über einen langen Zeitraum, zum Beispiel mindestens 1 Jahr, mindestens 5 Jahre, mindestens 10 Jahre oder mindestens 15 Jahre, zu gewährleisten. Der Stentgraft kann so ausgebildet sein, dass sein Durchmesser mit den intakten Gefäßbereichen im Verlauf des Herzrhythmus variieren kann. Gemäß einer bevorzugten Ausbildungsform ist der Stentgraft jedoch so ausgebildet, dass sein Durchmesser im Verlauf des Herzrhythmus nicht wesentlich variiert. Der Stentgraft ist bevorzugt metalllos. Sowohl Graft als auch Stentstruktur können biologisch abbaubar sein, wobei der Graft und die Stentstruktur mit gleichen oder unterschiedlichen Raten biologisch abbaubar sein können. In einer bevorzugten Ausführungsform des erfindungsgemäßen Stentgrafts sind jedoch Graft und Stentstruktur biologisch nicht abbaubar. Ferner kann der Stentgraft auch mehrere, gleiche oder verschiedene, Stentstrukturen und/oder Grafts aufweisen und/oder weitere Komponenten umfassen, die auf die mindestens eine Stentstruktur und/oder den mindestens einen Graft aufgebracht sind, wie beispielsweise eine oder mehrere Beschichtungen. Derartige Komponenten können biologisch abbaubar sein und/oder biokompatible Materialien aufweisen und/oder damit beschichtet sein. Der Stentgraft kann eine oder mehrere Beschichtungen aufweisen, wie Silikon, Kollagen und/oder Gelatine.

Der erfindungsgemäße Stentgraft kann auf verschiedene Arten in das betreffende Gefäß eingesetzt werden, insbesondere endovaskulär und/oder minimalinvasiv. So kann der Stentgraft bei der Einführung in das betroffene Gefäß den komprimierten Zustand aufweisen, der vorzugsweise einen geringeren Querschnitt als das betroffene Gefäß sowie gegebenenfalls weitere, während der Einführung des Stentgrafts zu passierende Gefäße aufweist. Der mindestens eine komprimierte Zustand weist dabei einen Durchmesser von 1 bis 20 mm auf, bevorzugt von 2 bis 15 mm und besonders bevorzugt 4 bis 10 mm. Ein geringerer Querschnitt kann insbesondere dadurch erreicht werden, dass die Querschnittsfläche um mehr als 30 %, bevorzugt um mehr als 50 %, und besonders bevorzugt um mehr als 80 % im Vergleich zu dem mindestens einen expandierten Zustand reduziert wird. An der Zielstelle kann der Stentgraft frei- und/oder eingesetzt werden, gefolgt von einer bevorzugt reversiblen Zustandsänderung in den expandierten Zustand, wobei der expandierte Zustand einen Durchmesser aufweist, der in etwa dem Durchmesser des intakten Gefäßes in dem betroffenen Bereich entspricht. Der mindestens eine expandierte Zustand weist damit einen Durchmesser von 0,5 cm bis 6 cm auf, bevorzugt von 0,7 cm bis 5 cm und besonders bevorzugt von 1 cm bis 4 cm. Der mindestens eine expandierte Zustand kann einen Durchmesser von mindestens 0,5 cm, mindestens 1 cm, mindestens 2 cm oder mindestens 3 cm aufweisen.

Der Stentgraft kann, beispielsweise abhängig von der individuellen Gefäßgeometrie, entlang seiner Längserstreckung einen konstanten Durchmesser aufweisen oder aber einen sich in Strömungsrichtung verjüngenden Durchmesser.

Der Übergang des Stentgrafts vom komprimierten in den expandierten Zustand kann durch die Freisetzung aus einem den Durchmesser begrenzenden Kathetersystem und/oder durch Ballondilatation erfolgen. Alternativ oder zusätzlich kann dieser durch einen externen Faktor ausgelöst werden, beispielsweise Feuchtigkeit, pH Wert, Lichtintensität, elektrische Felder, Magnetfelder und/oder insbesondere durch eine dauerhafte und/oder mindestens kurzzeitige Änderung der Temperatur. Eine solche dauerhafte und/oder mindestens kurzzeitige Temperaturänderung kann beispielsweise passiv durch den Unterschied zwischen Raumtemperatur und der im Gefäß herrschenden Temperatur realisiert werden. So wird die Änderung von dem mindestens einen komprimierten Zustand in den mindestens einen expandierten Zustand bevorzugt durch eine kurzzeitige Erhöhung der Temperatur auf Werte zwischen 30 °C und 60 °C, bevorzugt zwischen 33 °C und 50 °C und besonders bevorzugt zwischen 35 °C und 40 °C ausgelöst. Es kann sich hierbei um eine Erhöhung der Umgebungstemperatur aufgrund der Körpertemperatur selbst handeln. Eine Änderung der Temperatur kann zusätzlich und/oder alternativ auch aktiv ausgelöst werden, beispielweise durch Spülung eines Kathetersystems und/oder des Gefäßes mit temperierter Kochsalzlösung, z. B. um eine verfrühte Zustandsänderung während der Einführung und Positionierung des Stentgrafts zu vermeiden.

Der Stentgraft ist vorzugsweise so ausgebildet, dass er über eine hinreichende Länge an der Gefäßwand, insbesondere einem intakten Abschnitt der Gefäßwand, anliegt und dadurch an der gewünschten Position lagefest ist. Zu diesem Zweck kann der Stentgraft zum Beispiel eine Länge von 1 cm bis 35 cm aufweisen, bevorzugt jedoch und zwischen 1 cm und 30 cm besonders bevorzugt zwischen 5 cm und 25 cm. Die Länge des Stentgrafts kann mindestens 3 cm, mindestens 4 cm oder mindestens 5 cm betragen. Die Länge des Stentgrafts kann geringer als 30 cm, geringer als 20 cm oder geringer als 15 cm sein.

Für eine lagefeste Positionierung können in Endbereichen an den mindestens zwei offenen Enden der hohlzylindrischen Form des Stentgrafts, mindestens jedoch in dem Endbereich des in Strömungsrichtung aufwärts gelegenen Endes, Kontaktflächen zur Anlage an der intakten Gefäßwand ausgebildet sein, den sogenannten Landungszonen. Die jeweilige Kontaktfläche weist bevorzugt eine Länge zwischen 0,1 cm bis 5 cm, bevorzugt zwischen 0,5 cm bis 2 cm auf. Optional kann der Stentgraft auch Haken oder anderweitige Elemente zur Verankerung und/oder Positionierung des Stentgrafts in dem betroffenen Bereich aufweisen, zum Beispiel an dem in Strömungsrichtung aufwärts gelagerten Ende. Hierdurch kann eine besonders starke Verankerung erreicht werden.

Routinemäßig verwendete Stentgrafts sind in der Regel verallgemeinerte Standardprodukte. Diese benötigen in der Regel für eine lagefeste Positionierung in unmittelbarer Nähe des Bereichs der veränderten Gefäßwand eine intakte und durchgehende Gefäßwand mit einer Länge von mindestens 10 bis 15 mm. Befindet sich die Gefäßwandveränderung jedoch an oder nahe einer Verzweigung, kann die Verwendung solcher Standardprodukte ausgeschlossen sein, da diese die Blutversorgung des abzweigenden Gefäßes unterbrechen würden. Bauchaortenaneurysmen (AAA) oder Aneurysmen der thorakalen Aorta (TAA) sowie Aneurysmen, die über beide oben genannten Bereiche hinweg gehen (thorakoabdominale Aortenaneurysmen (TAAA)) entstehen häufig in der Nähe solcher Gefäßverzweigungen, wie beispielsweise der Nierenarterien. Der Stentgraft kann daher zusätzlich zu den offenen Enden seiner tubulären Struktur mit weiteren Öffnungen ausgebildet sein, die gegebenenfalls auch aufweitbar ausgestaltet sein können.

Der bereitgestellte Graft kann zur Ausbildung dieser Öffnungen eine oder mehrere Scallops und/oder Fenestrierungen aufweisen. Der Begriff Scallop bezeichnet eine Aussparung an einem Ende des bevorzugt tubulären und/oder tubusförmigen Grafts. Bevorzugt bezeichnet der Begriff Scallop hierbei eine drei-, bevorzugt viereckige oder auch U-förmige Aussparung am in Strömungsrichtung aufwärts gelegenen Ende des Grafts. Ein Scallop weist hierbei eine in Strömungsrichtung gemessene Aussparungshöhe von 3 mm bis 20 mm auf, bevorzugt von 5 mm bis 15 mm, sowie eine Weite (vorzugsweise quer zur Strömungsrichtung oder in Umfangsrichtung gemessen) von 2 mm bis 15 mm, bevorzugt von 7 mm bis 12 mm. Der Begriff Fenestrierung bezeichnet sogenannte Fenster, offene Zonen oder Löcher im Graft die nach korrekter Positionierung des Stentgrafts in dem betroffenen Bereich hinsichtlich Position und Durchmesser mit einem jeweiligen abzweigenden Gefäß in etwa deckungsgleich sind. Die Fenestrierungen sind bevorzugt oval oder rund ausgestaltet, besonders bevorzugt mit einem Verhältnis von Höhe zu Breite zwischen 0,5 und 2,5, bevorzugt zwischen 0,8 und 2, besonders bevorzugt zwischen 1 und 1,35. Ferner können Scallops und/oder Fenestrierungen patientenindividuell gestaltet sein.

Der Graft kann insbesondere in Fällen, in denen der Bereich der Gefäßwandveränderung eine Verbindung zu einem weiteren Gefäß aufweist und/oder die Distanz zwischen dem Bereich der Gefäßwandveränderung und einer Verbindung zu einem weiteren Gefäß geringer als 10 mm ist, eine oder mehrere Fenestrierungen und/oder Scallops aufweisen. Die Anzahl der Fenestrierungen kann zwischen 0 und 10, vorzugsweise zwischen 1 und 5 liegen. Die Fenestrierungen weisen vorzugsweise einen Durchmesser zwischen 1 mm und 15 mm, besonders bevorzugt zwischen 3 mm und 12 mm oder zwischen 4 mm und 8 mm auf. Der Durchmesser der einen oder der mehreren Fenestrierungen ist vorzugsweise mindestens 2 mm, mindestens 4 mm oder mindestens 5 mm.

Die Stentstruktur wird vorzugsweise so auf dem Graft vorgesehen, dass die Streben die Fenestrierung bzw. die Fenestrierungen und/oder Scallops nicht kreuzen und/oder den Blutfluss durch diese nicht beeinträchtigen.

Kleinere Fenestrierungen, etwa mit einem Durchmesser von beispielsweise 5 mm bis 8 mm, können erfindungsgemäß zwischen Streben der Stentstruktur im Graft vorgesehen sein. Insofern kann ein Muster, insbesondere ein regelmäßiges Muster (etwa regelmäßig in Umfangsrichtung; z. B. ein Mäandermuster mit einer bestimmten Amplitude und/oder einem bestimmten Intervall) der Stentstruktur im Bereich der jeweiligen Fenestrierung regelmäßig und/oder unverändert sein. So kann etwa die Amplitude und/oder das Intervall des Musters der Stentstruktur im Bereich der Fenestrierung unverändert bleiben. Erfindungsgemäße Verfahren können einen Schritt des Ausrichtens der aufzubringenden Stentstruktur mit der Fenestrierung im Graft aufweisen. Hierbei kann es sich insbesondere um ein virtuelles Ausrichten handeln, das vorzugsweise vor Herstellung der Stentstruktur erfolgt, etwa in einem CAD-Programm (Computer Aided Design) und/oder einem CAM-Programm (Computer Aided Manufacturing).

Gegebenenfalls können Fenestrierungen von Streben gekreuzt werden, etwa große Fenestrierungen im Graft mit einem Durchmesser von beispielsweise 8 mm bis 15 mm. Vorzugsweise kann jedoch eine Anpassung der Stentstruktur - insbesondere des Strebenmusters - so erfolgen, dass ein Kreuzen und/oder eine Beeinträchtigung einer lichten Öffnung der Fenestrierung vermieden oder zumindest weitestgehend vermieden wird. Die Stentstruktur und/oder die Streben der Stentstruktur können hierbei um die Fenestrierung herum geführt sein, etwa bei einem zickzack- oder mäanderförmigen Strebenmuster indem die Amplitude und/oder das Intervall des Musters im Bereich der Fenestrierung geändert (insbesondere vergrößert) wird.

Alternativ oder zusätzlich kann die Anordnung der Streben dahingehend ausgelegt sein, dass Ränder der Fenestrierung von Streben gestützt werden. So können die Streben beispielsweise zirkulär und/oder oval (etwa ellipsoid) zumindest teilweise (etwa über einen Winkel von > 90°, > 180° oder > 270°) oder vollständig um eine jeweilige Fenestrierung herum verlaufen.

Fenestrierungen und/oder Scallops können beispielsweise mittels Stanzen, Lochen, Ausschneiden, Laserschneiden, Aufweiten und/oder mit Hilfe von beispielsweise Brennscheren bereitgestellt werden. Bevorzugt weist der bereitgestellte Graft jedoch eine oder mehrere Fenestrierungen und/oder Scallops auf, die bereits bei seiner Herstellung bereitgestellt wurden (etwa durch ein entsprechendes Weben oder Wirken des Grafts). So kann eine nachträgliche lokale Änderung der Materialeigenschaften und/oder ein Durchtrennen eines oder mehrere der Fäden, die den Graft bilden, vermieden werden. Dies ist zeiteffizient, da keine Nachbearbeitung des Grafts benötigt wird und keine potentiellen Schwachstellen im Graft erzeugt werden. Die Fäden des Graft sind im Bereich der Fenestrierungen und/oder Scallops daher vorzugsweise nicht durchtrennt und/oder verlaufen um diese Fenestrierungen und/oder Scallops herum.

Der Stentgraft kann optional an mindestens einem Ende und/oder mindestens einer Fenestrierung verzweigt sein, wobei z. B. eine Stentstruktur und/oder ein Graftabschnitt in ein abzweigendes Blutgefäß hineinragt ("Bifurcation" beziehungsweise "Branch"). Der abzweigende Abschnitt kann mindestens 0,5 cm, mindestens 1 cm oder mindestens 3 cm lang sein. Dies kann dazu beitragen, eine lagefeste Position einer Fenestrierung zu gewährleisten und/oder den Bereich zwischen Graft und abgehendem Gefäß zu überbrücken, zu verbinden und/oder diesen abzudichten. Alternativ und/oder zusätzlich kann der Stentgraft auch mit mindestens einer weiteren Stentstruktur und/oder einem weiteren Stentgraft in Kontakt stehen, der in einem mit dem betroffenen Gefäß in Verbindung stehenden weiteren Gefäß positioniert ist oder wird. Im Fall eines solchen modularen Aufbaus kann der Kontakt beispielsweise durch partielle Überlappung realisiert werden.

Der Stentgraft kann als standardisiertes oder auch als individualisiertes Produkt hergestellt werden. Insbesondere im Fall von Fenestrierungen und/oder Verzweigungen im und/oder nahe des betroffenen Bereichs kann es vorteilhaft sein, den Stentgraft individualisiert für das betroffene Gefäß herzustellen, da Ausbildung und Lage der Verzweigung stark variieren können. Bevorzugt wird der Stentgraft an Hand von Bilddaten des betroffenen Gefäßes individualisiert ausgestaltet. Insbesondere kann die Struktur und/oder die Form des Grafts (etwa die Lage, Form und/oder Größe der einen oder der mehreren Fenestrierungen) und/oder die Struktur, die Form und/oder das Muster der Stentstruktur (etwa der Verlauf der Streben und/oder ihr Querschnitt) basierend auf solchen Bilddaten patientenindividualisiert ausgestaltet und/oder hergestellt werden.

Informationen bezüglich der Lage des betroffenen Bereichs im Körper, der Ausdehnung des zu stützenden oder zu ersetzenden Gefäßabschnittes sowie möglicher, in oder nahe dem betroffenen Bereich liegender Gefäßabzweigungen können vor der Herstellung des Grafts und/oder vor der Aufbringung der Stentstruktur auf den Graft durch medizinische Bildgebung erhalten werden, insbesondere mittels Computer- und Magnetresonanztomographie, optional aber auch mittels Röntgen- oder Ultraschallvorrichtungen, vor allem wenn sie zur Aufnahme eines 3D-Bilddatensatzes geeignet sind. Als Grundlage für die Herstellung eines individualisierten Stentgrafts, weisen die Bilddaten vorzugsweise eine Schichtdicke von maximal 3 mm, bevorzugt von maximal 2 mm und besonders bevorzugt von maximal 1 mm auf. Die Daten werden vorzugsweise quer zur Strömungsrichtung in einem Abstand von 0,05 mm bis 10 mm, bevorzugt in einem Abstand von 0,5 mm bis 3 mm aufgenommen. Zwei aufeinanderfolgende Schichten können eine Überlappung von maximal 50%, bevorzugt von maximal 25% Überlappung aufweisen, wobei der Abstand zwischen den Schichten maximal so groß wie die entsprechende Schichtdicke ist. Um hochqualitative Informationen hinsichtlich der exakten Ausgestaltung der Gefäßwand zu gewährleisten, werden die gewonnenen und/oder erhaltenen Bilder bevorzugt auf eine dem Fachmann bekannte Art nachbearbeitet, beispielsweise mittels multiplanarer Rekonstruktion, um eine möglichst hohe räumliche Auflösung in alle Raumrichtungen bereitzustellen, bevor sie zur Herstellung eines individualisierten Stentgrafts herangezogen werden.

Die gewonnenen und/oder bereitgestellten Bilddaten können dabei zu einem oder mehreren Zeitpunkten erstellt worden sein und werden in einem bevorzugten erfindungsgemäßen Verfahren zur Herstellung eines computergestützten Modells des Grafts, der Stentstruktur und/oder des Stentgrafts herangezogen, um einen individualisierten Stentgraft auszugestalten.

Das bzw. die computergestützte Modell/e, insbesondere ein digitales 3D-Modell, kann hierbei Ausdehnung, 3D-Struktur und/oder Materialeigenschaften des Stentgrafts umfassen sowie Informationen bezüglich der Herstellung, beispielsweise welche Materialien in welchem Bereich des Stentgrafts auf welche Weise verarbeitet werden sollen. Dabei wird das Modell als digitale Darstellung bevorzugt mittels eines Computersystems und/oder einer CAD-Software in Querschnittschichten unterteilt, die zum Erstellen des Gefäßimplantats in einem Prozess der additiven Fertigung verwendet werden. Bevorzugter Weise ist das Verfahren zur Herstellung eines erfindungsgemäßen und an Hand von bereitgestellten Bilddaten individualisierten Stentgrafts voll automatisierbar. Das erfindungsgemäße Verfahren, insbesondere die direkte Aufbringung der Stentstruktur auf den Graft, ermöglicht dabei optimale Individualisierung bei zugleich stark verkürzter Produktionszeit.

Gemäß des erfindungsgemäßen Verfahrens können bei der Definition der Geometrie des Grafts (insbesondere bei Erstellung des computergestützten Modells des Grafts) geometrische Daten des Gefäßes herangezogen und insbesondere in ein computergestütztes Programm eingespeist werden. Alternativ oder zusätzlich können bei der Definition der Geometrie der Stentstruktur (insbesondere bei Erstellung des computergestützten Modells der Stentstruktur) geometrische Daten des Gefäßes und/oder geometrische Daten des Grafts herangezogen und insbesondere in ein computergestütztes Programm eingespeist werden. Das Verfahren kann z. B. einen Schritt umfassen, in dem geometrische Daten des Grafts und geometrische Daten der Stentstruktur (etwa die beiden computergestützten Modelle) in einem Programm zusammengeführt werden. Dies kann z. B. die Überlagerung der beiden computergestützten Modelle erlauben, z. B. in einem gemeinsamen computergestützten Modell und/oder einer gemeinsamen Ansicht.

Bevorzugte Ausführungsformen der Erfindung werden nachfolgend unter Bezugnahme auf die Zeichnungen beispielhaft beschrieben. Es handelt sich hierbei lediglich um schematische Darstellungen, die häufig zur Verdeutlichung bestimmter Aspekte andere (optionale) Strukturen nicht darstellen oder aber auch verschiedene optionale, miteinander einhergehende Aspekte in einer Darstellung berücksichtigen. Gleiche Bezugszeichen weisen in diesem Zusammenhang auf äquivalente, ähnliche, vergleichbare oder gleiche Bauteile in den dargestellten Ausführungsformen hin.

Die gezeigten Ausführungsformen können innerhalb des Schutzumfangs der Ansprüche in vielerlei Hinsicht verändert werden. Die Offenbarung der Figuren soll den Schutzumfang der Erfindung nicht beschränken. Dabei ist zu beachten, dass die Merkmale der obengenannten Ausführungsformen in einer einzigen Ausführungsform kombiniert werden können. Ausführungsformen der Erfindung können daher je nach Ausgestaltung alle oder nur einige der obengenannten Merkmale aufweisen. Es zeigen:
- Fig. 1: ein schematisches Ablaufdiagramm eines erfindungsgemäßen Verfahrens;
- Fig. 2: eine schematische Darstellung des erfindungsgemäßen Aufbringens der Stentstruktur auf einen Graft;
- Fig. 3: eine schematische Darstellung des erfindungsgemäßen Stentgrafts in einem komprimierten und einem expandierten Zustand.

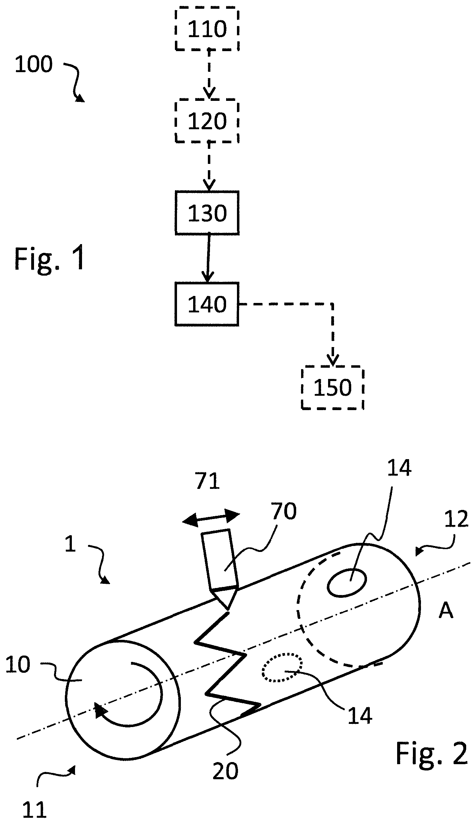

**Figur 1** zeigt in einem schematischen Ablaufdiagramm die Schritte eines erfindungsgemäßen Verfahrens **100** zur Herstellung eines Stentgraft **1** (siehe auch **Figur 2****),** wobei gestrichelte Linien optionale Schritte anzeigen. In einer bevorzugten Ausführungsform des Verfahrens werden zunächst pre-operative medizinische Bilddaten eines Gefäßes bereitgestellt (Schritt **110),** die beispielsweise im Zuge einer Computer- und/oder Magnetresonanztomographie gewonnen wurden. Die Bilddaten können auf dem Fachmann bekannte Weise bearbeitet werden, um eine möglichst hohe 3-dimensionale Auflösung zu erhalten, und zur Erstellung eines computergestützten Modells des Graft **10,** der Stentstruktur **20** und/oder des Stentgraft **1** herangezogen werden (Schritt **120).** Die Herstellung des erfindungsgemäßen und bevorzugt individualisierten Stentgraft **1** erfolgt durch das Bereitstellen (Schritt **130)** eines, vorzugsweise polymerbasierten, Graft **10,** auf den eine Stentstruktur **20** mit einer Mehrzahl von Streben aus einem vorzugsweise polymerbasierten Material aufgebracht wird (Schritt **140).** Dies geschieht vorzugsweise mittels Fused Deposition Modelling als additivem Fertigungsverfahren.

Der erfindungsgemäße Stentgraft 1 kann nach dem Aufbringen der Stentstruktur in Schritt **140,** optional umgestülpt werden (Schritt **150),** etwa um die Stentstruktur **20** innerhalb des Graft **10** anzuordnen.

**Figur 2** zeigt in perspektivischer Ansicht schematisch eine Herstellung des vorzugsweise tubulären und/oder tubusförmigen, insbesondere hohlzylindrischen Stentgraft **1,** der sich vorzugsweise von einem ersten offenen Ende **11** zu einem gegenüberliegenden, zweiten offenen Ende **12** erstreckt. Der vorzugsweise textilbasierte oder aus Textil bestehende Graft **10** kann auf einen Halter (nicht dargestellt) geschoben werden und/oder auf diesem aufliegen. Der Halter kann rotierbar gelagert sein, etwa sodass der Graft **10** um seine Längsachse **A** rotierbar ist. Die Geometrie des Halters kann entsprechend der Geometrie des Graft **10** gewählt werden, insbesondere kann der Außendurchmesser eines zylindrischen Halters entsprechend dem zu behandelnden Gefäßdurchmesser ausgewählt sein.

Der hier beispielhaft dargestellte tubuläre Graft **10** weist zwei Fenestrierungen **14** auf, die vorliegend im Bereich des zweiten Endes **12** gezeigt sind. Diese können jedoch auch an anderen Stellen des Graft entlang der Längsachse **A** vorgesehen sein (z. B. mittig). Zusätzlich und/oder alternativ könnte der Graft **10** zudem mindestens einen Scallop, weitere Fenestrierungen, und/oder Abzweigungen, so genannte Seitenarmstents ("Branches"), entlang der Längsachse **A** aufweisen. Ferner kann der Graft **10** zusätzlich und/oder alternativ weitere Öffnungen an den Enden **11, 12** aufweisen bzw. er könnte an einem dieser Enden **11, 12** verzweigt sein ("Bifurcation"; nicht gezeigt).

Die Stentstruktur **20** wird vorzugsweise direkt auf den Graft **10** aufgebracht, indem dafür vorgesehenes polymerbasiertes oder polymeres Material in einem beheizbaren Düsenkopf **70** erhitzt wird und ein oder mehrere verflüssigte Filamente in einer oder mehreren Schichten auf den Graft **10** aufgebracht werden **140.** Dies kann beispielsweise in einem automatisierten Verfahren nach einem in Schritt **120** erzeugten computergestützten Modell des Stentgrafts **1** realisiert werden, während der Düsenkopf **70** sich im Wesentlichen entlang der Längsrichtung des Grafts **10** bewegt (siehe Pfeil **71)** und der Halter bei Bedarf rotiert. Alternativ kann auch der Halter im Wesentlichen in Längsrichtung bewegt werden oder auch Halter und Düsenkopf **70.** Die so ausgebildete Mehrzahl miteinander verbundener Streben der Stentstruktur **20** kann optional als Ringe, Zickzack, Helix, Spiralen, Maschen und/oder sonstigen Geometrie angeordnet sein, wobei beispielsweise einzelne Segmente der Stentstruktur direkt oder indirekt über Verbinder miteinander verbunden sein können.

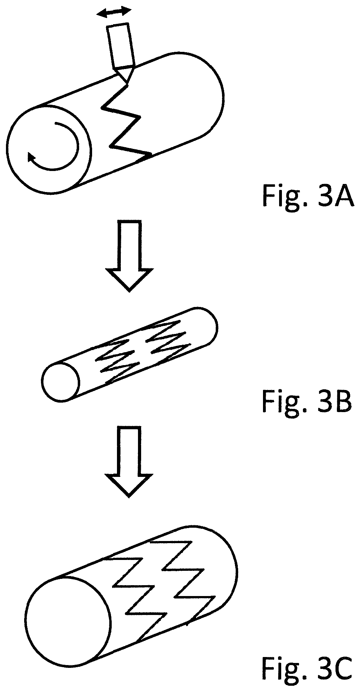

Der Stentgraft **10** kann auf bekannte Weise in ein Gefäß eingesetzt werden. **Figur 3** zeigt eine schematische Übersicht über verschiedene Zustände, die der Stentgraft mindestens aufweisen kann, wobei die Zustandsänderung auf Grund des Materials und/oder der Materialkombination der Stentstruktur bevorzugt reversibel und schnell erfolgt. Bei seiner Herstellung kann der Stentgraft zunächst einen ersten Zustand aufweisen **(****Fig. 3A****).** So kann der Stentgraft etwa im expandierten Zustand hergestellt werden. Nach seiner Herstellung, spätestens aber vor seiner bevorzugt endovaskulären und/oder minimalinvasiven Einführung in das betroffene Gefäß weist der Stentgraft einen komprimierten, gecrimpten Zustand auf **(****Fig. 3B****).** Hierfür kann je nach Stentgeometrie beispielsweise der Öffnungswinkel zwischen zwei an einem Verbindungs- und/oder Kreuzungspunkt miteinander verbundenen Streben verringert werden. Nachdem der Stentgraft in dem betroffenen Gefäßabschnitt in Position gebracht wurde, wird er in einen expandierten Zustand überführt, in dem er einen größeren Durchmesser als in dem mindestens einen gecrimpten Zustand aufweist **(****Fig. 3C****),** wobei der expandierte Zustand optional dem ersten Zustand entsprechen kann. Diese Zustandsänderung kann durch mechanische Krafteinwirkung bewirkt werden oder aber auf eine vordefinierte Art durch die Änderung eines externen Faktors, wenn ein entsprechendes Smart Polymer zur Ausbildung der Stentstruktur verwendet wurde (etwa durch ein Zurückspringen in eine expandierte Form bei Erhöhung der Temperatur). In diesem Fall wird der expandierte Zustand bevorzugt durch das Erreichen der Körpertemperatur und damit einer Temperatur von 37°C erreicht.

Die Stentstruktur weist im expandierten Zustand bevorzugt eine radiale Steifigkeit auf, die für eine Überbrückung oder Ersetzung des betroffenen Gefäßabschnitts ausreichend ist. Der Graft ist bevorzugt als selbstdichtendes System ausgestaltet, das zunächst flüssigkeitsdurchlässig ist und sich nach Implantation des Stentgrafts innerhalb kurzer Zeit durch Koagulation in der im Gefäß transportierten Flüssigkeit enthaltener Bestandteile und/oder deren Ablagerung in und/oder an dem Graft abdichtet.

Soweit die vorgehende Beschreibung den Ausdruck "im Wesentlichen" oder entsprechende Terme verwendet, sind auch solche Ausführungsformen umfasst, in denen das jeweilige Merkmal vollständig oder komplett vorliegt. Die Wörter "Vielzahl" oder "mehrere" sind im Sinne von "mindestens zwei", d.h., zwei oder mehr, zu verstehen. Sofern konkrete Werte angegeben werden, umfassen diese vorzugsweise auch geringfügige Abweichungen von diesen Werten, wie etwa Abweichungen von +/- 10% oder +/- 5% des jeweiligen Werts.

## Patentansprüche

1. Verfahren (100) zur Herstellung eines Stentgrafts (1), das folgende Schritte aufweist:
Bereitstellen (130) eines Graft (10) aus einem ersten polymerbasierten Material; und
Aufbringen (140) einer Stentstruktur (20) mit einer Mehrzahl von Streben aus einem zweiten polymerbasierten Material auf den Graft (10) mittels eines additiven Fertigungsverfahrens, vorzugsweise mittels Fused Deposition Modeling;
wobei der Graft (10) und die aufgebrachte Stentstruktur (20) so ausgebildet sind, dass der Stentgraft (1) in mindestens einem komprimierten Zustand und in mindestens einem expandierten Zustand anordenbar ist, wobei der Stentgraft (1) in dem mindestens einen komprimierten Zustand einen geringeren Querschnitt als in dem mindestens einen expandierten Zustand aufweist.

2. Verfahren (100) nach Anspruch 1, wobei der Graft (10) tubulär und/oder tubusförmig ist und wobei der Graft (10) optional Durchmessersprünge, Verzweigungen und/oder Abzweigungen aufweist.

3. Verfahren (100) nach Anspruch 1 oder 2, wobei der hergestellte Stentgraft (1) so ausgebildet ist, dass der Graft (10) innerhalb der Stentstruktur (20) angeordnet ist.

4. Verfahren (100) nach einem der vorherigen Ansprüche,
wobei der Graft (10) textil und/oder textilbasiert ist, insbesondere ein Gewebe und/oder eine Maschenware ist; und/oder
wobei der Graft (10) mittels Jacquard-Technik hergestellt ist; und/oder
wobei der Graft (10) nach seiner Herstellung eine Materialschichtdicke von 0,01 mm bis 2 mm aufweist.

5. Verfahren (100) nach einem der vorherigen Ansprüche,
wobei die Stentstruktur (20) ein Smart Polymer, insbesondere ein Shape Memory Polymer, und/oder ein nicht-smartes, biokompatibles Polymer aufweist; und/oder
wobei der Stentgraft (1) metalllos ist.

6. Verfahren (100) nach einem der vorherigen Ansprüche, wobei der bereitgestellte Graft (10) Fenestrierungen (14) aufweist, wobei die Fenestrierungen (14) vorzugsweise einen Durchmesser zwischen 1 mm und 15 mm, stärker bevorzugt zwischen 3 mm und 12 mm, besonders bevorzugt zwischen 4 mm und 8 mm aufweisen.

7. Verfahren (100) nach einem der vorherigen Ansprüche, wobei der bereitgestellte Graft (10) eine Vielzahl von durch die Maschenstruktur gebildeten Poren mit einem Durchmesser von 1 bis 1000 µm aufweist, und/oder wobei einzelne Poren eine Öffnungsfläche von 1 bis 1.000.000 µm² aufweisen.

8. Verfahren (100) nach einem der vorherigen Ansprüche,
wobei die Streben in einem Querschnitt senkrecht zur Verlaufsrichtung der jeweiligen Strebe in Radialrichtung des Stentgrafts (1) eine Höhe von mindestens 10 µm haben und/oder in dem Querschnitt senkrecht zur Höhe mindestens 10 µm, vorzugsweise mindestens 50 µm breit sind; und/oder
wobei die Streben in einem Querschnitt senkrecht zur Verlaufsrichtung der jeweiligen Strebe in Radialrichtung des Stentgrafts (1) aus mehreren Lagen aufgebaut sind; und/oder
wobei die Streben in einem Querschnitt senkrecht zur Verlaufsrichtung der jeweiligen Strebe senkrecht zur Radialrichtung des Stentgrafts (1) aus mehreren Schichten aufgebaut sind.

9. Verfahren (100) nach einem der vorherigen Ansprüche, das ferner die Schritte aufweist:
Erhalten (110) pre-operativer medizinischer Bilddaten eines Gefäßes; und
Erstellen (120) eines computergestützten Modells des Grafts (10), der Stentstruktur (20) und/oder des Stentgrafts (1) anhand der Bilddaten.

10. Verfahren (100) nach einem der vorherigen Ansprüche, wobei das Aufbringen der Stentstruktur (20) auf den Graft (10) durch den Einsatz eines rotierbaren Halters, mittels dem der Graft (10) gehalten und gedreht werden kann, erfolgt.

11. Verfahren (100) nach einem der vorherigen Ansprüche, wobei die Streben eine kompressible Helikale-, Zickzack- oder Maschenstruktur ausbilden und/oder mäanderförmige Ringe.

12. Verfahren (100) nach einem der vorherigen Ansprüche, wobei der Graft (10) eine oder mehrere Scallops und/oder Fenestrierungen aufweist.

13. Verfahren (100) nach einem der vorherigen Ansprüche,
wobei der bereitgestellte Graft (10) flüssigkeitsdurchlässig ausgestaltet ist und wobei der Graft (10) vorzugsweise ein selbstdichtendes System bildet; oder
wobei der bereitgestellte Graft (10) flüssigkeitsundurchlässig ist, vorzugsweise
wobei der bereitgestellte Graft (10) flüssigkeitsundurchlässig ist durch eine Beschichtung, wobei das Verfahren (100) einen Schritt einer Beschichtung des Grafts (10) vor dem Aufbringen einer Stentstruktur (20) auf den Graft (10) aufweist, oder
wobei der bereitgestellte Graft (10) flüssigkeitsundurchlässig ist durch eine Beschichtung, wobei das Verfahren (100) nach dem Aufbringen einer Stentstruktur (20) auf den Graft (10) einen Schritt einer Beschichtung des hergestellten Stentgrafts (1) aufweist.

14. Verfahren (100) nach einem der vorherigen Ansprüche, wobei der Stentgraft (1) mindestens eine Stentstruktur (20) aufweist, die zumindest teilweise innen und außen auf dem Graft (10) angeordnet ist, und/oder wobei der Stentgraft (1) mindestens eine Stentstruktur (20) aufweist, die auf einer Innenseite des Grafts (10) angeordnet ist, und mindestens eine Stentstruktur (20), die auf einer Außenseite des Grafts (10) angeordnet ist.

15. Stentgraft (1), hergestellt nach dem Verfahren (100) gemäß einem der vorherigen Ansprüche, wobei vorzugsweise die Stentstruktur (20) den Graft (10) umgibt.

## Claims

1. A method (100) for producing a stent graft (1), which comprises the following steps:
providing (130) a graft (10) made of a first polymer-based material; and
applying (140) a stent structure (20) comprising a plurality of struts made of a second polymer-based material onto the graft (10) by means of an additive method, preferably by means of fused deposition modeling;
wherein the graft (10) and the applied stent structure (20) are configured such that the stent graft (1) is arrangeable in at least one compressed state and in at least one expanded state, wherein the stent graft (1) has a smaller cross-section in the at least one compressed state than in the at least one expanded state.

2. The method (100) according to claim 1, wherein the graft (10) is tubular and/or tube-shaped and wherein the graft (10) optionally comprises diameter changes, bifurcations and/or branches.

3. The method (100) according to claim 1 or 2, wherein the produced stent graft (1) is configured such that the graft (10) is arranged within the stent structure (20).

4. The method (100) according to any one of the preceding claims,
wherein the graft (10) is textile and/or textile-based, in particular a weave and/or knitwear; and/or
wherein the graft (10) is produced by means of a jacquard technique; and/or
wherein the graft (10) has a material layer thickness of 0.01 mm to 2 mm after its production.

5. The method (100) according to any one of the preceding claims,
wherein the stent structure (20) comprises a smart polymer, in particular a shape memory polymer, and/or a non-smart, biocompatible polymer; and/or
wherein the stent graft (1) is metal-free.

6. The method (100) according to any one of the preceding claims, wherein the provided graft (10) comprises fenestrations (14), wherein the fenestrations (14) preferably have a diameter between 1 mm and 15 mm, more preferably between 3 mm and 12 mm, particularly preferably between 4 mm and 8 mm.

7. The method (100) according to any one of the preceding claims, wherein the provided graft (10) comprises a plurality of pores formed by the mesh structure and having a diameter of 1 to 1,000 µm, and/or wherein individual pores have an opening area of 1 to 1,000,000 µm².

8. The method (100) according to any one of the preceding claims,
wherein the struts, in a cross-section perpendicular to the direction in which the respective strut extends, have a height of at least 10 µm in the radial direction of the stent graft (1) and/or a width perpendicular to the height of at least 10 µm, preferably at least 50 µm, in the cross-section; and/or
wherein the struts, in a cross-section perpendicular to the direction in which the respective strut extends, in the radial direction of the stent graft (1), are made from a plurality of layers; and/or
wherein the struts, in a cross-section perpendicular to the direction in which the respective strut extends, perpendicular to the radial direction of the stent graft (1), comprise a plurality of layers.

9. The method (100) according to any one of the preceding claims, which further comprises the steps of:
obtaining (110) preoperative medical image data of a vessel; and
generating (120) a computer-aided model of the graft (10), the stent structure (20) and/or the stent graft (1) by means of the image data.

10. The method (100) according to any one of the preceding claims, wherein the stent structure (20) is applied onto the graft (10) by using a rotatable holder by means of which the graft (10) can be held and rotated.

11. The method (100) according to any one of the preceding claims, wherein the struts configure a compressible helical, zigzag or mesh structure and/or meander-shaped rings.

12. The method (100) according to any one of the preceding claims, wherein the graft (10) comprises one or more scallops and/or fenestrations.

13. The method (100) according to any one of the preceding claims,
wherein the provided graft (10) is configured to be permeable to liquid and wherein the graft (10) preferably forms a self-sealing system; or
wherein the provided graft (10) is impermeable to liquid,
preferably
wherein the provided graft (10) is impermeable to liquid by means of a coating, wherein the method (100) comprises a step of coating the graft (10) before the application of a stent structure (20) onto the graft (10), or
wherein the provided graft (10) is impermeable to liquid by means of a coating, wherein, after the application of a stent structure (20) onto the graft (10), the method (100) comprises a step of coating the produced stent graft (1).

14. The method (100) according to any one of the preceding claims, wherein the stent graft (1) comprises at least one stent structure (20) that is arranged on the graft (10) at least partially inside and outside, and/or wherein the stent graft (1) comprises at least one stent structure (20) that is arranged on an inner side of the graft (10) and at least one stent structure (20) that is arranged on an outer side of the graft (10).

15. A stent graft (1) produced according to the method (100) according to any one of the preceding claims, preferably wherein the stent structure (20) surrounds the graft (10).

## Revendications

1. Procédé (100) pour la fabrication d'une endoprothèse couverte (1), qui présente les étapes suivantes :
la fourniture (130) d'un greffon (10) composé d'un premier matériau à base de polymère ; et
l'application (140) d'une structure d'endoprothèse (20) avec une pluralité d'entretoises composées d'un deuxième matériau à base de polymère sur le greffon (10) au moyen d'un procédé de fabrication additive, de préférence au moyen d'un dépôt de filament fondu ;
dans lequel le greffon (10) et la structure d'endoprothèse (20) appliquée sont réalisés de sorte que l'endoprothèse couverte (1) peut être disposée dans au moins un état comprimé et dans au moins un état déployé, dans lequel l'endoprothèse couverte (1) dans l'au moins un état comprimé présente une section transversale plus petite que dans l'au moins un état déployé.

2. Procédé (100) selon la revendication 1, dans lequel le greffon (10) est tubulaire et/ou en forme de tube et dans lequel le greffon (10) présente éventuellement des diamètres de saut, des bifurcations et/ou des ramifications.

3. Procédé (100) selon la revendication 1 ou 2, dans lequel l'endoprothèse couverte (1) fabriquée est réalisée de sorte que le greffon (10) est disposé à l'intérieur de la structure d'endoprothèse (20).

4. Procédé (100) selon l'une quelconque des revendications précédentes,
dans lequel le greffon (10) est textile et/ou à base de textile, en particulier est un tissu et/ou un tricot ; et/ou
dans lequel le greffon (10) est fabriqué au moyen d'une technique Jacquard ; et/ou
dans lequel le greffon (10) après sa fabrication présente une épaisseur de matériau de 0,01 mm à 2 mm.

5. Procédé (100) selon l'une quelconque des revendications précédentes,
dans lequel la structure d'endoprothèse (20) présente un polymère intelligent, en particulier un polymère à mémoire de forme, et/ou un polymère biocompatible, non intelligent ; et/ou
dans lequel l'endoprothèse couverte (1) est exempte de métal.

6. Procédé (100) selon l'une quelconque des revendications précédentes, dans lequel le greffon (10) fourni présente des fenestrations (14), dans lequel les fenestrations (14) présentent de préférence un diamètre compris entre 1 mm et 15 mm, plus préférentiellement entre 3 mm et 12 mm, de manière particulièrement préférée entre 4 mm et 8 mm.

7. Procédé (100) selon l'une quelconque des revendications précédentes, dans lequel le greffon (10) fourni présente une pluralité de pores formés par la structure maillée avec un diamètre de 1 à 1000 µm, et/ou dans lequel différents pores présentent une surface d'ouverture de 1 à 1 000 000 µm².

8. Procédé (100) selon l'une quelconque des revendications précédentes,
dans lequel les entretoises possèdent dans une section transversale perpendiculairement à la direction d'étendue de l'entretoise respective dans la direction radiale de l'endoprothèse couverte (1) une hauteur d'au moins 10 µm et/ou dans la section transversale perpendiculairement à la hauteur une largeur d'au moins 10 µm, de préférence d'au moins 50 µm ; et/ou
dans lequel les entretoises dans une section transversale perpendiculairement à la direction d'étendue de l'entretoise respective dans la direction radiale de l'endoprothèse couverte (1) sont constituées de plusieurs couches ; et/ou
dans lequel les entretoises dans une section transversale perpendiculairement à la direction d'étendue de l'entretoise respective perpendiculairement à la direction radiale de l'endoprothèse couverte (1) sont constituées de plusieurs couches.

9. Procédé (100) selon l'une quelconque des revendications précédentes, qui présente en outre les étapes :
d'obtention (110) de données d'image médicales préopératoires d'un vaisseau ; et
d'élaboration (120) d'un modèle assisté par ordinateur du greffon (10), de la structure d'endoprothèse (20) et/ou de l'endoprothèse couverte (1) en s'appuyant sur les données d'image.

10. Procédé (100) selon l'une quelconque des revendications précédentes, dans lequel l'application de la structure d'endoprothèse (20) sur le greffon (10) s'effectue à l'aide d'un support rotatif, au moyen duquel le greffon (10) peut être maintenu et amené en rotation.

11. Procédé (100) selon l'une quelconque des revendications précédentes, dans lequel les entretoises réalisent une structure hélicoïdale, en zigzag ou maillée compressible et/ou des anneaux sinueux.

12. Procédé (100) selon l'une quelconque des revendications précédentes, dans lequel le greffon (10) présente une ou plusieurs dentelures et/ou fenestrations.

13. Procédé (100) selon l'une quelconque des revendications précédentes,
dans lequel le greffon (10) fourni est conçu de manière perméable au liquide et dans lequel le greffon (10) forme de préférence un système auto-étanche ; ou
dans lequel le greffon (10) fourni est imperméable au liquide, de préférence
dans lequel le greffon (10) fourni est imperméable au liquide du fait d'un revêtement, dans lequel le procédé (100) présente une étape d'un revêtement du greffon (10) avant l'application d'une structure d'endoprothèse (20) sur le greffon (10), ou
dans lequel le greffon (10) fourni est imperméable au liquide du fait d'un revêtement, dans lequel le procédé (100) après l'application d'une structure d'endoprothèse (20) sur le greffon (10) présente une étape d'un revêtement de l'endoprothèse couverte (1) fabriquée.

14. Procédé (100) selon l'une quelconque des revendications précédentes, dans lequel l'endoprothèse couverte (1) présente au moins une structure d'endoprothèse (20), qui est disposée au moins en partie à l'intérieur et à l'extérieur sur le greffon (10), et/ou dans lequel l'endoprothèse couverte (1) présente au moins une structure d'endoprothèse (20), qui est disposée sur un côté intérieur du greffon (10), et au moins une structure d'endoprothèse (20), qui est disposée sur un côté extérieur du greffon (10).

15. Endoprothèse couverte (1), fabriquée selon le procédé (100) selon l'une quelconque des revendications précédentes, dans lequel de préférence la structure d'endoprothèse (20) entoure le greffon (10).
